# EUROPEAN PATENT SPECIFICATION

(11) **EP 1 796 642 B1**
(45) Date of publication and mention of the grant of the patent: **21.05.2008**
(21) Application number: 05791660.3
(22) Date of filing: 29.08.2005
(51) Int. Cl.: A61K 9/14

(54) **PHARMACEUTICAL COMPOSITIONS IN THE FORM OF SOLID DISPERSIONS FOR THE TREATMENT OF CANCER**
PHARMAZEUTISCHE ZUSAMMENSETZUNGEN IN FORM FESTER DISPERSIONEN ZUR BEHANDLUNG VON KREBS
COMPOSITIONS PHARMACEUTIQUES SOUS FORME DE DISPERSIONS SOLIDES POUR LE TRAITEMENT DU CANCER

(30) Priority: 27.08.2004 US 604753 P
(43) Date of publication of application: 20.06.2007
(73) Proprietor: Bayer Pharmaceuticals Corporation, West Haven, CT 06516-4175 (US)
(72) Inventor: SCHUECKLER, Fritz, 51467 Bergisch Gladbach (DE)
(74) Representative: Albers, Markus
(86) International application number: PCT/US2005/030542
(87) International publication number: WO 2006/026501

(56) References cited:
- WO-A-03/047523
- WO-A-03/068228
- US-A1- 2003 144 278
- US-A1- 2003 207 872

## Description

### Field of the Invention

This invention relates to novel pharmaceutical compositions, to processes for preparing these novel pharmaceutical compositions and to their use for treating hyper-proliferative disorders, such as cancer, either as a sole agent or in combination with other therapies.

### Background of the Invention

Diarylureas are a class of serine-threonine kinase inhibitors as well as tyrosine kinase inhibitors known in the art. The following publications illustrate their utility as active ingredient in pharmaceutical compositions for the treatment of hyper-proliferative diseases, such as cancer:
Smith et al., Bioorg. Med Chem. Lett. 2001, 11, 2775-2778.
Lowinger et al., Clin. Cancer Res. 2000, 6(suppl.), 335.
Lyons et al., Endocr.-Relat. Cancer 2001, 8, 219-225.
Riedl et al., Book of Abstracts, 92nd AACR Meeting, New Orleans, LA, USA, abstract 4956*.*
Khire et al., Book of Abstracts, 93rdAACR Meeting, San Francisco, CA, USA, abstract 4211*.*
Lowinger et al., Curr. Pharm. Design 2002, 8, 99-110.
Carter et al., Book of Abstracts, 92ndAACR Meeting, New Orleans, LA, USA, abstract 4954*.*
Vincent et al., Book of Abstracts, 38th ASCO Meeting, Orlando, FL, USA, abstract 1900*.*
Hilger et al., Book of Abstracts, 38th ASCO Meeting, Orlando, FL, USA, abstract 1916*.*
Moore et al., Book of Abstracts, 38th ASCO Meeting, Orlando, FL, USA, abstract 1816*.*
Strumberg et al., Book of Abstracts, 38th ASCO Meeting, Orlando, FL, USA, abstract 121*.*
Omega-Carboxyaryl diphenyl ureas are disclosed in WO00/42012 (published July 20, 2000), WOOO/41698 (published July 20, 2000), and in the following published U.S. applications:
   US2002-0165394-A1, published November 7, 2002,
   US2001-003447-A1, published October 25, 2001,
   US2001-0016659-A1, published August 23, 2001,
   US2002-013774-A1, published September 26, 2002,
   and copending U.S. applications:
   09/758,547, filed January 12, 2001,
   09/889,227, filed July 12, 2001,
   09/993,647, filed November 27, 2001,
   10/042,203, filed January 11, 2002 and
   10/071,248, filed February 11, 2002

In particular, it has been discovered that the diphenyl urea of Formula I, also referred as "BAY 43-9006" or 4{4-[3-(4-chloro-3-trifluoromethylphenyl}-ureido]-phenoxy}-pyridine-2-carboxylic acid methyl amide, is a potent inhibitor of raf, VEGFR-2, p38, and PDGFR kinases. These enzymes are all molecular targets of interest for the treatment of hyper-proliferative diseases, including cancer. Therefore, the compound of Formula I will be used as medicine for the treatment of the above mentioned diseases.

The following publications describe the urea of formula I and pharmaceutical compositions containing it: WO-03/068228, US 2003/144278, US 2003/207872, WO 03/047523.

A preferred route of drug administration is through the oral cavity. This route provides great comfort and convenience of dosing. The bioavailability achieved after oral administration is a measure for the potential usefulness of an oral dosage form of a drug. Bioavailability after oral application depends on several factors, such as solubility of the active in aqueous media, dose strength, dissolution of the dosage form, absorption throughout the gastrointestinal tract and first pass effect.
Therefore solid pharmaceutical compositions for oral application containing the compound of Formula I, which result in improved dissolution, absorption and exposure in mammals, improved inter-patient variability, and overall improved efficacy in the clinic are desired.

### Description of the Invention

Pharmaceutical compositions comprising a solid dispersion of the compound of Formula I below are provided.

Formula I is as follows:

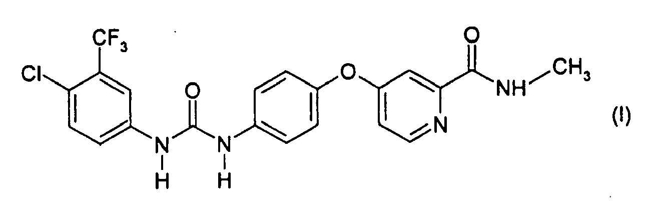

The term "the compound of Formula I", or "the compound of this invention" does not only refer to 4{4-[3-(4-chloro-3-trifluoromethylphenyl)-ureido]-phenoxy}-pyridine-2-carboxylic acid methyl amide as depicted in Formula I, but also refers to its solvates, hydrates, pharmaceutically acceptable salts, or a combination thereof.

The present invention pertains to
(i) novel pharmaceutical compositions containing the compound of Formula I in the form of a solid dispersion, which includes solid solutions, glass solutions, glass suspensions, amorphous precipitations in a crystalline carrier, eutectics or monotecics, compound or complex formation and combinations thereof,
(ii) processes for preparing these novel pharmaceutical compositions, and
(iii) the use of these compositions for the treatment of hyper-proliferative diseases, such as cancer, either as a sole agent, or in combination with other anti-cancer therapies.

In the following, the different types of solid dispersions (solid solutions, glass solutions, glass suspensions, amorphous precipitations in a crystalline carrier, eutectics or monotecics, compound or complex formation and combinations thereof) are collectively referred to as "solid dispersion."

A pharmaceutical composition according to this invention comprises of a solid dispersion comprising at least the compound of Formula I and a pharmaceutically acceptable matrix.

The term "matrix" or "matrix agents" as used herein refers to both polymeric excipients, non-polymeric excipients and combinations thereof, capable of dissolving or dispersing the compound of formula I.

An aspect of the invention of particular interest is a pharmaceutical composition comprising a solid dispersion, wherein the matrix comprises a pharmaceutically acceptable polymer, such as polyvinylpyrrolidone, vinylpyrrolidone/vinylacetate copolymer, polyalkylene glycol (i.e. polyethylene glycol), hydroxyalkyl cellulose (i.e. hydroxypropyl cellulose), hydroxyalkyl methyl cellulose (i.e. hydroxypropyl methyl cellulose), carboxymethyl cellulose, sodium carboxymethyl cellulose, ethyl cellulose, polymethacrylates, polyvinyl alcohol, polyvinyl acetate, vinyl alcohol/vinyl acetate copolymer, polyglycolized glycerides, xanthan gum, carrageenan, chitosan, chitin, poyldextrin, dextrin, starch, proteins or combinations thereof.

Another aspect of the invention is a pharmaceutical composition comprising a solid dispersion, wherein the matrix comprises a sugar and/or sugar alcohol and/or cyclodextrin, for example sucrose, lactose, fructose, maltose, raffinose, sorbitol, lactitol, mannitol, maltitol, erythritol, inositol, trehalose, isomalt, inulin, maltodextrin, β-cyclodextrin, hydroxypropyl-β-cyclodextrin, sulfobutyl ether cyclodextrin or combinations thereof.

An aspect of the invention of particular interest is a pharmaceutical composition comprising a solid dispersion which is an amorphous co-precipitate of a compound of Formula I and polyvinylpyrrolidone.

Additional suitable excipients that are useful in the formation of the matrix of the solid dispersion include, but are not limited to alcohols, organic acids, organic bases, amino acids, phospholipids, waxes, salts, fatty acid esters, polyoxyethylene sorbitan fatty acid esters, and urea.

The solid dispersion of the compound of Formula I in the matrix may contain certain additional pharmaceutical acceptable ingredients, such as carriers, surfactants, fillers, disintegrants, recrystallization inhibitors, plasticizers, defoamers, antioxidants, detackifier, pH-modifiers, glidants and lubricants.

A carrier according to this invention is an excipient, which becomes loaded with a mixture, comprised of at least the matrix agent and the compound of this invention, during the manufacturing process of the solid dispersion, for example by hot melt extrusion, hot melt coating, prilling, congealing, solvent evaporation processes (e.g. layering, coating, granulation), and thus becomes an integral part of the solid dispersion.

In an embodiment of this invention, the matrix comprises a water soluble polymer.

In another embodiment, at least one from the group of polyvinylpyrrolidone, copovidone, hydroxypropyl cellulose, hydroxypropyl methyl cellulose, polyethylene glycol and polyethylene oxide is used as matrix agent in the solid dispersion.

In another embodiment, polyvinylpyrrolidone is used as matrix agent.

Another embodiment comprises hydroxypropyl cellulose as matrix agent.

Another aspect of the invention of particular interest are solid dispersions containing croscarmellose sodium, sodium starch glycollate, crospovidone, low substituted hydroxypropyl cellulose (L-HPC), starch, microcrystalline cellulose or a combination thereof as carrier or disintegrant.

In an embodiment, the solid dispersion comprises polyvinylpyrrolidone and croscarmellose sodium.

In another embodiment, the solid dispersion comprises polyvinylpyrrolidone and sodium starch glycollate.

In another embodiment, the solid dispersion comprises polyvinylpyrrolidone, croscarmellose sodium and microcrystalline cellulose.

In another embodiment, the solid dispersion comprises hydroxypropyl cellulose and croscarmellose sodium.

In yet another embodiment, the solid dispersion comprises hydroxypropyl cellulose and at least one excipient, which is a sugar, sugar alcohol, cyclodextrin or combination thereof.

The solid dispersion of the invention is prepared according to methods known to the art for the manufacture of solid dispersions, such as fusion/melt technology, hot melt coating, prilling, congealing, solvent evaporation (e.g. freeze drying, spray drying, vacuum drying, layering of powders of granules, powders or pellets an fluid bed granulation), coprecipitation, supercritical fluid technology and electrostatic spinning method.

Hot melt extrusion or solvent evaporation techniques are suitable processes for preparation of solid dispersion formulations of this invention.

A solvent suitable for manufacture of solid dispersions by solvent evaporation processes such as spray-drying, layering and fluid-bed granulation can be any compound, wherein the compound of Formula I can be dissolved. Preferred solvents include alcohols (e.g. methanol, ethanol, n-propanol, isopropanol, and butanol), ketones (e.g. acetone, methyl ethyl ketone and methyl isobutyl ketone), esters (e.g. ethyl acetate and propyl acetate) and various other solvents such as acetonitrile, methylene chloride, choroform, hexane, toluene, tetrahydrofuran, cyclic ethers, and 1,1,1-trichloroethane. Lower volatility solvents, such as dimethyl acetamide or dimethylsulfoxide can also be used. Mixtures of solvents, can also be used, as can mixtures with water as long as the drug and if necessary the matrix agent are sufficiently soluble to make the process practicable.

An aspect of the invention of particular interest is a pharmaceutical composition in which the compound of Formula I is substantially amorphous.

Another aspect of the invention of particular interest is a solid dispersion of the compound of Formula I, wherein the matrix is a polyvinylpyrrolidone polymer.

Another aspect of the invention of particular interest is a solid dispersion of the compound of Formula I, wherein the matrix is a hydroxypropylcellulose polymer.

Another aspect of this invention which is of particular interest is a novel pharmaceutical composition comprising a co-precipitate of a compound of formula I and a matrix.

This pharmaceutical composition will be utilized to achieve the desired pharmacological effect by oral administration to a patient in need thereof, and will be advantageous to a conventional formulation in terms of drug release, bioavailability, and/or interpatient variability in mammals. A patient, for the purpose of this invention, is a mammal, including a human, in need of treatment for the particular condition or disease, including prophylactic treatment.

For oral administration, the solid dispersion described herein can be formulated into solid or liquid preparations such as powder, granules, pellets, tablets, capsules, dragées, chewable tablets, dispersible tables, troches, lozenges, melts, solutions, suspensions, or emulsions, and may be prepared according to methods known to the art for the manufacture of pharmaceutical compositions. For this purpose the solid dispersion may be compounded with conventional excipients, for example binders, fillers, lubricants, disintegrants, solvents, surfactants, thickeners and stabilizers, coating materials as well as flavoring agents, sweeteners, flavoring and coloring agents.

It is believed that one skilled in the art, utilizing the preceding information, can utilize the present invention to its fullest extent. The oral formulation of the compound of Formula I refers to a wide range of dosages such as 1 mg, 10 mg, 100 mg, or even 1 g daily dosing and beyond. This would be accomplished, for example, by modifying the composition and size of the tablet or capsule, and/or by administering multiple tablets or capsules per day to the patient in need thereof. Alternatively, the solid dispersion formulation may also be dosed in forms such as powders, granules, chewable or dispersible tablets, or by dispersions of any adequate solid formulation in a suitable liquid prior to use, for example if the optimal dose regimen was no longer consistent with a feasible tablet or capsule size.

### Method of treating hyper-proliferative disorders

The present invention also relates to a method for using a new oral pharmaceutical composition of the compound of Formula I to treat mammalian hyper-proliferative disorders, including cancer: This method comprises administering the pharmaceutical composition in the form of a solid dispersion to a mammal in need thereof, including a human, an amount which is effective to treat the disorder. The term "hyper-proliferative disorders" and/or "cancer" not only refers to solid tumors, such as cancers of the breast, respiratory tract, brain, reproductive organs, digestive tract, urinary tract, eye, liver, skin, head and neck, thyroid, parathyroid and their distant metastases, but also includes lymphomas, sarcomas, and leukemias.

Examples of breast cancer include, but are not limited to invasive ductal carcinoma, invasive lobular carcinoma, ductal carcinoma *in situ,* and lobular carcinoma in situ.

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypophtalmic glioma, cerebellar and cerebral astrocytoma, medulloblastoma, ependymoma, as well as neuroectodermal and pineal tumor.

Tumors of the male reproductive organs include, but are not limited to prostate and testicular cancer. Tumors of the female reproductive organs include, but are not limited to endometrial, cervical, ovarian, vaginal, and vulvar cancer, as well as sarcoma of the uterus.

Tumors of the digestive tract include, but are not limited to anal, colon, colorectal, esophageal, gallbladder, gastric, pancreatic, rectal, small intestine, and salivary gland cancers.

Tumors of the urinary tract include, but are not limited to bladder, penile, kidney, renal pelvis, ureter, and urethral cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal / hypopharyngeal / nasopharyngeal / oropharyngeal cancer, and lip and oral cavity cancer.

Lymphomas include, but are not limited to AIDS-related lymphoma, non-Hodgkin's lymphoma, cutaneous T-cell lymphoma, Hodgkin's disease, and lymphoma of the central nervous system.

Sarcomas include, but are not limited to sarcoma of the soft tissue, fibrosarcoma, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, and can be treated by administering the pharmaceutical compositions of the present invention.

The total amount of the active ingredient (compound of Formula I) to be administered via the oral route using the pharmaceutical composition of the present invention will generally range from about 0.01 mg/kg to about 50 mg/kg body weight per day. Based upon standard laboratory techniques known to evaluate compounds useful for the treatment of hyper-proliferative disorders, by standard toxicity tests and by standard pharmacological assays for the determination of treatment of the conditions identified above in mammals, and by comparison of these results with the results of known medicaments that are used to treat these conditions, the effective dosage of the pharmaceutical compositions of this invention can readily be determined by those skilled in the art. The amount of the administered active ingredient can vary widely according to such considerations as the particular compound and dosage unit employed, the mode and time of administration, the period of treatment, the age, sex, and general condition of the patient treated, the nature and extent of the condition treated, the rate of drug metabolism and excretion, the potential drug combinations and drug-drug interactions, and the like.

The pharmaceutical compositions of this invention can be administered as the sole agent or in combination with one or more other therapies where the combination causes no unacceptable adverse effects. For example, they can be combined with cytotoxic agents, signal transduction inhibitors, or with other anti-cancer agents or therapies, as well as with admixtures and combinations thereof.

In one embodiment, the pharmaceutical compositions of the present invention can be combined with cytotoxic anti-cancer agents. Examples of such agents can be found in the 11th Edition of the Merck Index (1996). These agents include, by no way of limitation, asparaginase, bleomycin, carboplatin, carmustine, chlorambucil, cisplatin, colaspase, cyclophosphamide, cytarabine, dacarbazine, dactinomycin, daunorubicin, doxorubicin (adriamycine), epirubicin, etoposide, 5-fluorouracil, hexamethylmelamine, hydroxyurea, ifosfamide, irinotecan, leucovorin, lomustine, mechlorethamine, 6-mercaptopurine, mesna, methotrexate, mitomycin C, mitoxantrone, prednisolone, prednisone, procarbazine, raloxifen, streptozocin, tamoxifen, thioguanine, topotecan, vinblastine, vincristine, and vindesine.

Other cytotoxic drugs suitable for use with the pharmaceutical compositions of the invention include, but are not limited to, those compounds acknowledged to be used in the treatment of neoplastic diseases in Goodman and Gilman's The Pharmacological Basis of Therapeutics (Ninth Edition, 1996, McGraw-Hill). These agents include, by no way of limitation, aminoglutethimide, L-asparaginase, azathioprine, 5-azacytidine cladribine, busulfan, diethylstilbestrol, 2', 2'-difluorodeoxycytidine, docetaxel, erythrohydroxynonyladenine, ethinyl estradiol, 5-fluorodeoxyuridine, 5-fluorodeoxyuridine monophosphate, fludarabine phosphate, fluoxymesterone, flutamide, hydroxyprogesterone caproate, idarubicin, interferon, medroxyprogesterone acetate, megestrol acetate, melphalan, mitotane, paclitaxel, pentostatin, N-phosphonoacetyl-L-aspartate (PALA), plicamycin, semustine, teniposide, testosterone propionate, thiotepa, trimethylmelamine, uridine, and vinorelbine.

Other cytotoxic anti-cancer agents suitable for use in combination with the compositions of the invention also include newly discovered cytotoxic principles such as oxaliplatin, gemcitabine, capecitabine, epothilone and its natural or synthetic derivatives, temozolomide (Quinn et al., J. Clin. Oncology 2003, 21(4), 646-651), tositumomab (Bexxar), trabedectin (Vidal et al., Proceedings of the American Society for Clinical Oncology 2004, 23, abstract 3181), and the inhibitors of the kinesin spindle protein Eg5 (Wood et al., Curr. Opin. Pharmacol. 2001, 1, 370-377).

In another embodiment, the pharmaceutical compositions of the present invention can be combined with other signal transduction inhibitors. Of particular interest are signal transduction inhibitors which target the EGFR family, such as EGFR, HER-2, and HER-4 (Raymond et al., Drugs 2000, 60 (Suppl.1), 15-23; Harari et al., Oncogene 2000, 19 (53), 6102-6114), and their respective ligands. Examples of such agents include, by no way of limitation, antibody therapies such as Herceptin (trastuzumab), Erbitux (cetuximab), and pertuzumab. Examples of such therapies also include, by no way of limitation, small-molecule kinase inhibitors such as ZD-1839 / Iressa (Baselga et al., Drugs 2000, 60 (Suppl. 1), 33-40), OSI-774 / Tarceva (Pollack et al. J. Pharm. Exp. Ther. 1999, 291(2), 739-748), CI-1033 (Bridges, Curr. Med. Chem. 1999, 6, 825-843), GW-2016 (Lackey et al., 92nd AACR Meeting, New Orleans, March 24-28, 2001, abstract 4582*),* CP-724,714 (Jani et al., Proceedings of the American Society for Clinical Oncology 2004, 23, abstract 3122), HKI-272 (Rabindran et al., Cancer Res. 2004, 64, 3958-3965), and EKB-569 (Greenberger et al., 11th NCI-EORTC-AACR Symposium on New Drugs in Cancer Therapy, Amsterdam, November 7-10, 2000, abstract 388*).*

In another embodiment, the pharmaceutical compositions of the present invention can be combined with other signal transduction inhibitors targeting receptor kinases of the split-kinase domain families (VEGFR, FGFR, PDGFR, flt-3, c-kit, c-fms, and the like), and their respective ligands. These agents include, by no way of limitation, antibodies such as Avastin (bevacizumab). These agents also include, by no way of limitation, small-molecule inhibitors such as STI-571 / Gleevec (Zvelebil, Curr. Opin. Oncol., Endocr. Metab. Invest. Drugs 2000, 2(1), 74-82), PTK-787 (Wood et al., Cancer Res. 2000, 60(8), 2178-2189), SU-11248 (Demetri et al., Proceedings of the American Society for Clinical Oncology 2004, 23, abstract 3001), ZD-6474 (Hennequin et al., 92nd AACR Meeting, New Orleans, March 24-28, 2001, abstract 3152*),* AG-13736 (Herbst et al., Clin. Cancer Res. 2003, 9, 16 (suppl 1), abstract C253), KRN-951 (Taguchi et al., 95th AACR Meeting, Orlando, FL, 2004, abstract 2575), CP-547,632 (Beebe et al., Cancer Res. 2003, 63, 7301-7309), CP-673,451 (Roberts et al., Proceedings of the American Association of Cancer Research 2004, 45, abstract 3989), CHIR-258 (Lee et al., Proceedings of the American Association of Cancer Research 2004, 45, abstract 2130), MLN-518 (Shen et al., Blood 2003, 102, 11, abstract 476), and AZD-2171 (Hennequin et al., Proceedings of the American Association of Cancer Research 2004, 45, abstract 4539).

In another embodiment, the pharmaceutical compositions of the present invention can be combined with inhibitors of the Raf/MEK/ERK transduction pathway (Avruch et al., Recent Prog. Horm. Res. 2001, 56, 127-155), or the PKB (akt) pathway (Lawlor et al., J. Cell Sci. 2001, 114, 2903-2910). These include, by no way of limitation, PD-325901 (Sebolt-Leopold et al., Proceedings of the American Association of Cancer Research 2004, 45, abstract 4003), and ARRY-142886 (Wallace et al., Proceedings of the American Association of Cancer Research 2004, 45, abstract 3891).

In another embodiment, the pharmaceutical compositions of the present invention can be combined with inhibitors of histone deacetylase. Examples of such agents include, by no way of limitation, suberoylanilide hydroxamic acid (SAHA), LAQ-824 (Ottmann et al., Proceedings of the American Society for Clinical Oncology 2004, 23, abstract 3024), LBH-589 (Beck et al., Proceedings of the American Society for Clinical Oncology 2004, 23, abstract 3025), MS-275 (Ryan et al., Proceedings of the American Association of Cancer Research 2004, 45, abstract 2452), and FR-901228 (Piekarz et al., Proceedings of the American Society for Clinical Oncology 2004, 23, abstract 3028).

In another embodiment, the pharmaceutical compositions of the present invention can be combined with other anti-cancer agents such as proteasome inhibitors, and m-TOR inhibitors. These include, by no way of limitation, bortezomib (Mackay et al., Proceedings of the American Society for Clinical Oncology 2004, 23, Abstract 3109), and CCI-779 (Wu et al., Proceedings of the American Association of Cancer Research 2004, 45, abstract 3 849).

Generally, the use of cytotoxic and/or cytostatic anti-cancer agents in combination with the pharmaceutical compositions of the present invention will serve to:
(1) yield better efficacy in reducing the growth of a tumor or even eliminate the tumor as compared to administration of either agent alone,
(2) provide for the administration of lesser amounts of the administered agents,
(3) provide for a chemotherapeutic treatment protocol that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time for tumor progression, and/or
(8) yield efficacy and tolerability results at least as good as those of the agents used alone, compared to known instances where other cancer agent combinations produce antagonistic effects.

It is believed that one skilled in the art, using the preceding information and information available in the art, can utilize the present invention to its fullest extent.

Example 1 refers to a preparation of the compound used in this invention. Representative solid dispersion formulations of the compound of this invention are described in Examples 2, 3, and 4.

### Examples

### Example 1: Preparation of 4{4-[3-(4-chloro-3-trifluoromethylphenyl)-ureido]-phenoxy}-pyridine-2-carboxylic acid methyl amide

A method of preparing BAY 43-9006 (4{4-[3-(4-chloro-3-trifluoromethylphenyl)-ureido]-phenoxy}-pyridine-2-carboxylic acid methyl amide) is described in Bankston et al. "A Scaleable Synthesis of BAY 43-9006: A Potent Raf Kinase Inhibitor for the Treatment of Cancer" Org. Proc. Res. Dev. 2002, 6(6), 777-781.

### Example 2: Preparation of 1:1, 1:2, 1:3, 1:4, and 1:5 solid dispersion of the compound of Example 1 with polyvinylpyrrolidone.

In an uncapped vial, one part of the compound of Example 1 as a free base was mixed with one, two, three, four, or five parts polyvinylpyrrolidone (PVP-25 / Kollidon 25) respectively. The mixture was dissolved in a sufficient amount of a 5 to 1 mixture of acetone and ethanol, until all powders were in solution. The uncapped vial was placed into a vacuum oven set at 40°C, and let dry for at least 24 hours.

The *in vitro* dissolution properties of the pharmaceutical compositions of Example 2 are compared with those of the free base of Example 1 as depicted in the dissolution profile below.

Based on this data, it can be assumed that the oral administration of the compound of the present invention, as a solid dispersion, will result in an improved absorption and bioavailability in humans, compared with a conventional formulation.

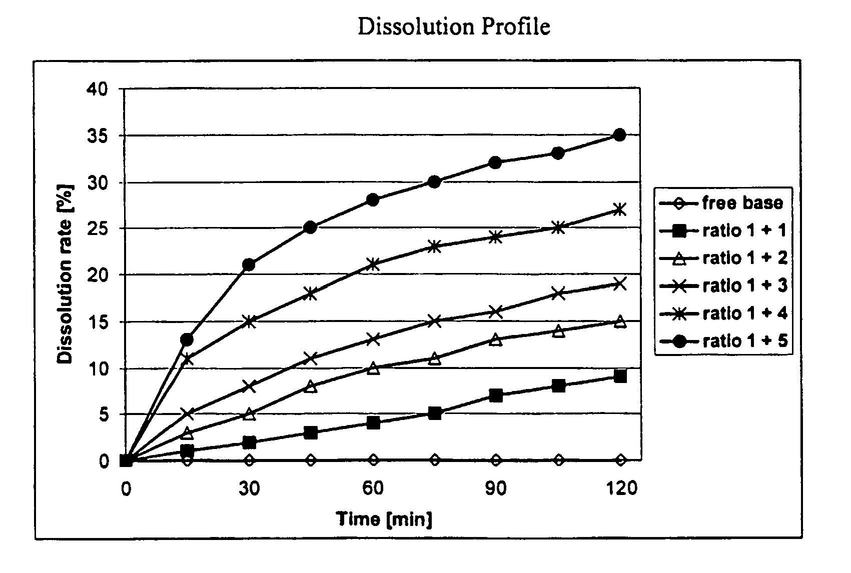

*In vitro* dissolution profiles of solid dispersions of the compound of the present invention in various amounts of polyvinylpyrrolidone (ratios ranging from 1:1 to 1:5).

### Example 3: Preparation of a 1:3 solid dispersion of the compound of Example 1 with hydroxypropyl cellulose.

In a heat-resistant vessel, one part of the compound of Example I as a free base was thoroughly mixed with three parts of hydroxypropyl cellulose (HPC-L), melted together at a temperature of > 200°C on a heating plate, then cooled back to room temperature.

### Example 4: Preparation of a 1:5 solid dispersion of the compound of Example 1 with starch and polyethylene glycol.

In a heat-resistant vessel, one part of the compound of Example 1 as a free base was thoroughly mixed with 4 parts of starch and one part of polyethylene glycol (PEG 6000), melted together at a temperature of > 200 °C on a heating plate, and then cooled to room temperature.

It is believed that this new type of pharmaceutical composition, comprising a solid dispersion of the compound of Formula I, will result in improved bioavailability, improved inter-patient variability, and overall superior efficacy for the treatment of hyper-proliferative diseases, including cancer.

Based on these findings it can be assumed that this new type of pharmaceutical composition, comprising a solid dispersion of the compound of Formula I, will result in improved absorption and exposure, reduced inter-patient variability, and overall superior efficacy for the treatment of hyper-proliferative disorders, including cancer.

## Claims

1. A composition containing 4{4-[3-(4-chloro-3-trifluoromethylphenylhureido]-phenoxy}-pyridine-2-carboxylic acid methyl amide and/or salts, hydrates, solvates thereof in the form of a solid dispersion.

2. A composition comprising a solid dispersion comprising at least the compound of Formula I and a pharmaceutically acceptable matrix.

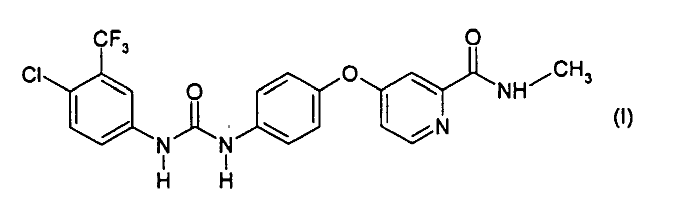

3. A composition according to claim 2, wherein the matrix comprises polymeric excipients or non-polymeric excipients capable of dissolving or dispersing the compound of Formula I.

4. A composition according to claim 2, wherein the matrix comprises a combination of polymeric excipients and non-polymeric excipients capable of dissolving or dispersing the compound of Formula I.

5. A composition according to claim 2, wherein the matrix comprises a water soluble polymer.

6. A compositions according to claim 5, wherein the matrix comprises at least one polymer from the group consisting of polyvinylpyrrolidone, copovidone, hydroxypropyl cellulose, hydroxypropyl methyl cellulose, polyethylene glycole or polyethylene oxide.

7. A composition according to claim 6, wherein the matrix comprises polyvinylpyrrolidone.

8. A composition according to claim 7, wherein the weight ratio of the compound of Formula I calculated as solvent-free base to polyvinylpyrrolidone is between 1:0.5 and 1:20.

9. A composition according to claim 6, wherein the matrix comprises hydroxypropyl cellulose.

10. A composition according to claim 9, wherein the weight ratio of the compound of Formula I calculated as solvent-free base to hydroxypropyl cellulose is between 1:0.5 and 1:20.

11. A composition according to any of the proceeding claims, wherein the solid dispersion comprises croscarmellose sodium, sodium starch glycolate, crospovidone, low substituted hydroxypropyl cellulose, starch, microcrystalline cellulose or a combination thereof.

12. A composition according to claim 8, wherein the solid dispersion comprises polyvinylpyrrolidone and croscarmellose sodium.

13. A composition according to claim 8, wherein the solid dispersion comprises polyvinylpyrrolidone and sodium starch glycolate.

14. A composition according to claim 8, wherein the solid dispersion comprises polyvinylpyrrolidone, croscarmellose sodium and microcrystalline cellulose.

15. A composition according to claim 10, wherein the solid dispersion comprises hydroxypropyl cellulose and croscarmellose sodium.

16. A composition according to claim 10, wherein the solid dispersion comprises hydroxypropyl cellulose and at least one excipient which is a sugar, sugar alcohol, cyclodextrin.

17. A composition according to any of the proceeding claims, wherein the solid dispersion is substantially homogeneous.

18. A composition according to any of the proceeding claims, which contains the compound of Formula I in substantially amorphous form.

19. A composition as claimed in any one of the proceeding claims, which is a pharmaceutical composition for oral application.

20. A composition as claimed in any one of the proceeding claims, which is a pharmaceutical composition in the form of a tablet.

21. A composition as claimed in any one of the proceeding claims, which is a pharmaceutical composition in the form of a capsule.

22. A composition as claimed in any one of the proceeding claims, which is a pharmaceutical composition in the form of a powder, granulate or sachet.

23. A process for the preparation of a solid dispersion of the compound of Formula I 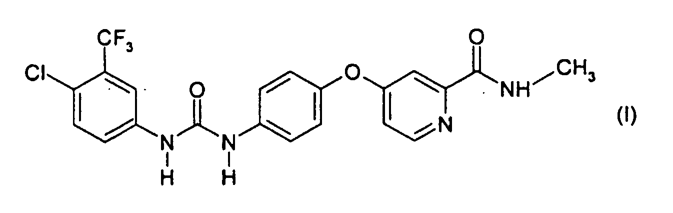 which comprises simultaneously exposing the compound of Formula I and at least one matrix to hot melt extrusion, hot melt coating, prilling, congealing, solvent evaporation techniques or a combination thereof.

24. A process according to claim 23, wherein the solid dispersion is prepared by exposing the compound of Formula I and at least one matrix agent to hot melt extrusion.

25. A process according to claim 23, wherein the solid dispersion is prepared by exposing the compound of Formula I and at least one matrix agent to solvent evaporation techniques.

26. A process as claimed in claim 23 to 25 wherein the solid dispersion is further treated with at least one additional processing step, which is milling, sieving, roller compaction, grinding, screening, mixing or a combination thereof.

27. A process as claimed in claim 23 to 26 comprising the additional step of compounding the solid dispersion with one or more pharmaceutical acceptable excipients to form a mixture and shaping this mixture into tablets, filled capsules or sachets.

28. A pharmaceutical composition, produced by a process of claims 23 to 27.

29. Use of a pharmaceutical composition according to any of the claims 1 to 22 and 28 for the manufacture of a medicament for treatment of hyper-proliferative disorders in a mammal, including a human, either as sole agent or in combination with other therapies.

30. Use of a pharmaceutical composition according to any of the claims 1 to 22 and 28 for the manufacture of a medicament for treatment of cancer in a mammal, including a human; either as sole agent or in combination with other therapies.

31. Use of a pharmaceutical composition according to any of the claims 1 to 22 and 28 for the manufacture of a medicament for treatment of cancer in a mammal, including a human, either as sole agent or in combination with radio therapy.

32. Use of a pharmaceutical composition according to any of the claims 1 to 22 and 28 for the manufacture of a medicament for treatment of cancer in a human patient, in combination with a cytotoxic therapy.

33. Use of a pharmaceutical composition according to any of the claims 1 to 22 and 28 for the manufacture of a medicament for treatment of cancer in a human patient, in combination with another anti-cancer therapy targeting either VEGFR, PDGFR, src, abl flt-3, EGFR, HER-2, aurora, raf, MEK, ERK, PI-3 kinase, AKT, mTOR, or HDAC.

## Patentansprüche

1. Zusammensetzung, die 4-{4-[3-(4-Chlor-3-trifluormethylphenyl)ureido]phenoxy}-pyridin-2-carbonsäuremethylamid und/oder Salze, Hydrate, Solvate davon in Form einer festen Dispersion enthält.

2. Zusammensetzung, umfassend eine feste Dispersion, die zumindest die Verbindung der Formel I und eine pharmazeutisch annehmbare Matrix umfasst.

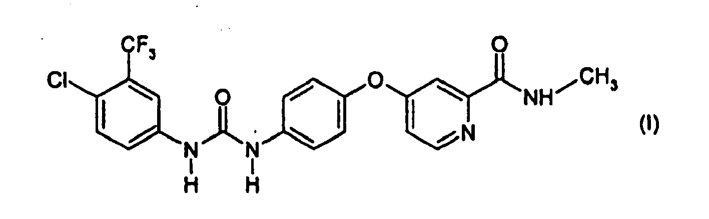

3. Zusammensetzung nach Anspruch 2, worin die Matrix polymere Arzneimittelträger oder nicht polymere Arzneimittelträger umfasst, die in der Lage sind, die Verbindung der Formel I zu lösen oder zu dispergieren.

4. Zusammensetzung nach Anspruch 2, worin die Matrix eine Kombination von polymeren Arzneimittelträgern und nicht polymeren Arzneimittelträgern umfasst, die in der Lage sind, die Verbindung der Formel I zu lösen oder zu dispergieren.

5. Zusammensetzung nach Anspruch 2, worin die Matrix ein wasserlösliches Polymer umfasst.

6. Zusammensetzungen nach Anspruch 5, worin die Matrix zumindest ein Polymer umfasst, das aus der aus Polyvinylpyrrolidon, Copovidon, Hydroxypropylcellulose, Hydroxypropylmethylcellulose, Polyethylenglykol und Polyethylenoxid bestehenden Gruppe ausgewählt ist.

7. Zusammensetzung nach Anspruch 6, worin die Matrix Polyvinylpyrrolidon umfasst.

8. Zusammensetzung nach Anspruch 7, worin das Gewichtsverhältnis der Verbindung der Formel I, berechnet als lösungsmittelfreie Base, zu Polyvinylpyrrolidon zwischen 1:0,5 und 1:20 beträgt.

9. Zusammensetzung nach Anspruch 6, worin die Matrix Hydroxypropylcellulose umfasst.

10. Zusammensetzung nach Anspruch 9, worin das Gewichtsverhältnis der Verbindung der Formel I, berechnet als lösungsmittelfreie Base, zu Hydroxypropylcellulose zwischen 1:0,5 und 1:20 beträgt.

11. Zusammensetzung nach einem der vorangegangenen Ansprüche, worin die feste Dispersion Croscarmellose-Natrium, Natriumstärkeglykolat, Crospovidon, niedrig substituierte Hydroxypropylcellulose, Stärke, mikrokristalline Cellulose oder eine Kombination davon umfasst.

12. Zusammensetzung nach Anspruch 8, worin die feste Dispersion Polyvinylpyrrolidon und Croscarmellose-Natrium umfasst.

13. Zusammensetzung nach Anspruch 8, worin die feste Dispersion Polyvinylpyrrolidon und Natriumstärkeglykolat umfasst.

14. Zusammensetzung nach Anspruch 8, worin die feste Dispersion Polyvinylpyrrolidon, Croscarmellose-Natrium und mikrokristalline Cellulose umfasst.

15. Zusammensetzung nach Anspruch 10, worin die feste Dispersion Hydroxypropylcellulose und Croscarmellose-Natrium umfasst.

16. Zusammensetzung nach Anspruch 10, worin die feste Dispersion Hydroxypropylcellulose und zumindest einen Arzneimittelträger umfasst, der ein Zucker, ein Zuckeralkohol oder Cyclodextrin ist.

17. Zusammensetzung nach einem der vorangegangenen Ansprüche, worin die feste Dispersion im Wesentlichen homogen ist.

18. Zusammensetzung nach einem der vorangegangenen Ansprüche, die die Verbindung der Formel I in im Wesentlichen amorpher Form enthält.

19. Zusammensetzung nach einem der vorangegangenen Ansprüche, die eine pharmazeutische Zusammensetzung zur oralen Anwendung ist.

20. Zusammensetzung nach einem der vorangegangenen Ansprüche, die eine pharmazeutische Zusammensetzung in Tablettenform ist.

21. Zusammensetzung nach einem der vorangegangenen Ansprüche, die eine pharmazeutische Zusammensetzung in Kapselform ist.

22. Zusammensetzung nach einem der vorangegangenen Ansprüche, die eine pharmazeutische Zusammensetzung in Form eines Pulvers, Granulats oder Beutels ist.

23. Verfahren zur Herstellung einer festen Dispersion der Verbindung der Formel I: 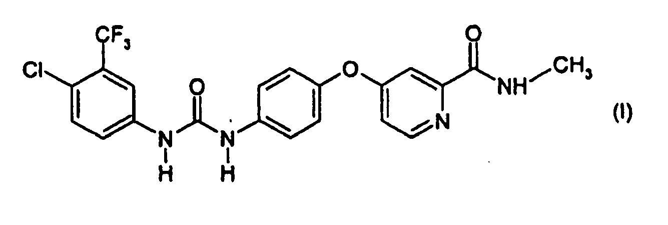 wobei die Verbindung der Formel I und zumindest eine Matrix zusammen Schmelzextrusions-, Schmelzbeschichtungs-, Prill-, Erstarrungs-, Lösungsmittelverdampfungsverfahren oder einer Kombination davon unterzogen werden.

24. Verfahren nach Anspruch 23, worin die feste Dispersion durch Schmelzextrudieren der Verbindung der Formel I und zumindest eines Matrixmittels hergestellt wird.

25. Verfahren nach Anspruch 23, worin die feste Dispersion hergestellt wird, indem die Verbindung der Formel I und zumindest ein Matrixmittel Lösungsmittelverdampfungsverfahren unterzogen werden.

26. Verfahren nach Anspruch 23 bis 25, worin die feste Dispersion in zumindest einem zusätzlichen Bearbeitungsschritt weiterbehandelt wird, wobei es sich um Mahlen, Sieben, Walzenverdichtung, Zerkleinerung, Filtern, Mischen oder eine Kombination davon handelt.

27. Verfahren nach Anspruch 23 bis 26, das den zusätzlichen Schritt des Kompoundierens der festen Dispersion mit einem oder mehreren pharmazeutisch annehmbaren Arzneimittelträgern zur Bildung eines Gemischs und des Formens dieses Gemischs zu Tabletten, gefüllten Kapseln oder Beuteln umfasst.

28. Pharmazeutische Zusammensetzung, hergestellt durch ein Verfahren nach einem der Ansprüche 23 bis 27.

29. Verwendung einer pharmazeutischen Zusammensetzung nach einem der Ansprüche 1 bis 22 und 28 zur Herstellung eines Medikaments zur Behandlung von Hyperproliferationsstörungen bei einem Säugetier, einschließlich eines Menschen, entweder als einziges Mittel oder in Kombination mit anderen Therapien.

30. Verwendung einer pharmazeutischen Zusammensetzung nach einem der Ansprüche 1 bis 22 und 28 zur Herstellung eines Medikaments zur Behandlung von Krebs bei einem Säugetier, einschließlich eines Menschen, entweder als einziges Mittel oder in Kombination mit anderen Therapien.

31. Verwendung einer pharmazeutischen Zusammensetzung nach einem der Ansprüche 1 bis 22 und 28 zur Herstellung eines Medikaments zur Behandlung von Krebs bei einem Säugetier, einschließlich eines Menschen, entweder als einziges Mittel oder in Kombination mit Strahlentherapie.

32. Verwendung einer pharmazeutischen Zusammensetzung nach einem der Ansprüche 1 bis 22 und 28 zur Herstellung eines Medikaments zur Behandlung von Krebs bei einem menschlichen Patienten in Kombination mit einer zytotoxischen Therapie.

33. Verwendung einer pharmazeutischen Zusammensetzung nach einem der Ansprüche 1 bis 22 und 28 zur Herstellung eines Medikaments zur Behandlung von Krebs bei einem menschlichen Patienten in Kombination mit einer anderen Anti-Krebs-Therapie, die auf VEGFR, PDGFR, scr, abl flt-3, EGFR, HER-2, Aurora, raf, MEK, ERK, PI-3-Kinase, AKT, mTOR oder HDAC abzielt.

## Revendications

1. Composition contenant du méthylamide d'acide 4-{4-[3-(4-chloro-3-trifluorométhylphényl)-uréido]-phénoxy}-pyridine-2-carboxylique et/ou ses sels, hydrates ou produits de solvatation, sous forme d'une dispersion solide.

2. Composition comprenant une dispersion solide qui comprend au moins le composé de formule I et une matrice pharmaceutiquement acceptable.

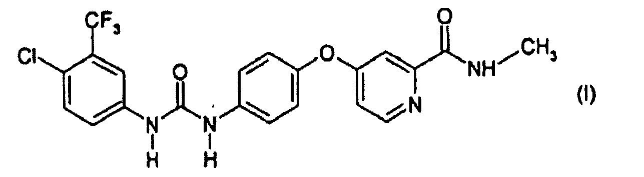

3. Composition suivant la revendication 2, dans laquelle la matrice comprend des excipients polymères ou des excipients non polymères capables de dissoudre ou disperser le composé de formule I.

4. Composition suivant la revendication 2, dans laquelle la matrice comprend une association d'excipients polymères et d'excipients non polymères capables de dissoudre ou disperser le composé de formule I.

5. Composition suivant la revendication 2, dans laquelle la matrice comprend un polymère hydrosoluble.

6. Compositions suivant la revendication 5, dans lesquelles la matrice comprend au moins un polymère du groupe consistant en polyvinylpyrrolidone, copovidone, hydroxypropylcellulose, hydroxypropylméthylcellulose, polyéthylèneglycol et poly(oxyde d'éthylène).

7. Composition suivant la revendication 6, dans laquelle la matrice comprend de la polyvinylpyrrolidone.

8. Composition suivant la revendication 7, dans laquelle le rapport pondéral du composé de formule I, calculé d'après la base sans solvant, à la polyvinylpyrrolidone est de 1:0,5 à 1:20.

9. Composition suivant la revendication 6, dans laquelle la matrice comprend de l'hydroxypropylcellulose.

10. Composition suivant la revendication 9, dans laquelle le rapport pondéral du composé de formule I, calculé d'après la base sans solvant, à l'hydroxypropylcellulose est de 1:0,5 à 1:20.

11. Composition suivant l'une quelconque des revendications précédentes, dans laquelle la dispersion solide comprend du croscarmellose sodique, du glycolate d'amidon sodique, de la crospovidone, une hydroxypropylcellulose à taux bas de substitution, de l'amidon, de la cellulose microcristalline ou leurs associations.

12. Composition suivant la revendication 8, dans laquelle la dispersion solide comprend de la polyvinylpyrrolidone et du croscarmellose sodique.

13. Composition suivant la revendication 8, dans laquelle la dispersion solide comprend de la polyvinylpyrrolidone et du glycolate d'amidon sodique.

14. Composition suivant la revendication 8, dans laquelle la dispersion solide comprend de la polyvinylpyrrolidone, du croscarmellose sodique et de la cellulose microcristalline.

15. Composition suivant la revendication 10, dans laquelle la dispersion solide comprend de l'hydroxypropylcellulose et du croscarmellose sodique.

16. Composition suivant la revendication 10, dans laquelle la dispersion solide comprend de l'hydroxypropylcellulose et au moins un excipient qui est un sucre, un sucre-alcool ou une cyclodextrine.

17. Composition suivant l'une quelconque des revendications précédentes, dans laquelle la dispersion solide est substantiellement homogène.

18. Composition suivant l'une quelconque des revendications précédentes, qui contient le composé de formule I sous une forme substantiellement amorphe.

19. Composition suivant l'une quelconque des revendications précédentes, qui est une composition pharmaceutique pour application orale.

20. Composition suivant l'une quelconque des revendications précédentes, qui est une composition pharmaceutique sous forme de comprimé.

21. Composition suivant l'une quelconque des revendications précédentes, qui est une composition pharmaceutique sous forme de capsule.

22. Composition suivant l'une quelconque des revendications précédentes, qui est une composition pharmaceutique sous forme d'une poudre, de granules ou d'un sachet.

23. Procédé pour la préparation d'une dispersion solide du composé de formule I 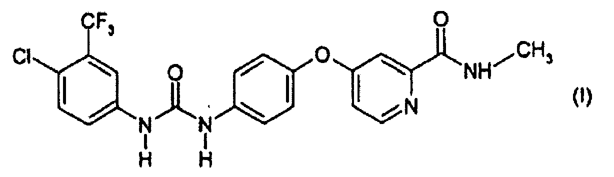 qui comprend simultanément l'exposition du composé de formule I et d'au moins une matrice à des techniques d'extrusion en masse fondue à chaud, d'enrobage en masse fondue à chaud, de pastillage, de figeage, d'évaporation de solvant ou à une de leurs associations.

24. Procédé suivant la revendication 23, dans lequel la dispersion solide est préparée en soumettant le composé de formule I et au moins un agent de matrice à une extrusion en masse fondue à chaud.

25. Procédé suivant la revendication 23, dans lequel la dispersion solide est préparée en soumettant le composé de formule I et au moins un agent de matrice à des techniques d'évaporation de solvant.

26. Procédé suivant les revendications 23 à 25, dans lequel la dispersion solide est en outre traitée par au moins une étape de traitement supplémentaire, qui est un broyage, un tamisage, un compactage au rouleau, une mouture, un criblage, un mélange ou une de leurs associations.

27. Procédé suivant les revendications 23 à 26, comprenant l'étape supplémentaire consistant à mélanger la dispersion solide à un ou plusieurs excipients pharmaceutiquement acceptables pour former un mélange et à façonner ce mélange en comprimés, capsules remplies ou sachets.

28. Composition pharmaceutique produite par un procédé selon les revendications 23 à 27.

29. Utilisation d'une composition pharmaceutique suivant l'une quelconque des revendications 1 à 22 et 28, pour la production d'un médicament destiné au traitement de troubles hyperprolifératifs chez un mammifère, y compris un être humain, comme seul agent ou en association avec d'autres thérapies.

30. Utilisation d'une composition pharmaceutique suivant l'une quelconque des revendications 1 à 22 et 28, pour la production d'un médicament destiné au traitement du cancer chez un mammifère, y compris un être humain, comme seul agent ou en association avec d'autres thérapies.

31. Utilisation d'une composition pharmaceutique suivant l'une quelconque des revendications 1 à 22 et 28, pour la production d'un médicament destiné au traitement du cancer chez un mammifère, y compris un être humain, comme seul agent ou en association avec la radiothérapie.

32. Utilisation d'une composition pharmaceutique suivant l'une quelconque des revendications 1 à 22 et 28, pour la production d'un médicament destiné au traitement du cancer chez un patient humain, en association avec une thérapie cytotoxique.

33. Utilisation d'une composition pharmaceutique suivant l'une quelconque des revendications 1 à 22 et 28, pour la production d'un médicament destiné au traitement du cancer chez un patient humain, en association avec une autre thérapie anticancéreuse ayant pour cible VEGFR, PDGFR, src, abl flt-3, EGFR, HER-2, aurora, raf, MEK, ERK, PI-3 kinase, AKT, mTOR ou HDAC.
